# EUROPEAN PATENT SPECIFICATION

(11) **EP 3 097 070 B1**
(45) Date of publication and mention of the grant of the patent: **05.08.2020**
(21) Application number: 15740632.3
(22) Date of filing: 24.01.2015
(51) Int. Cl.: C07C 7/12, C07C 7/09, B01D 3/14, B01D 3/40

(54) **ADSORPTION/DISTILLATION IN A SINGLE COLUMN DESIGN**
ADSORPTION/DESTILLATION IN EINEM EINZELSÄULENENTWURF
ADSORPTION/DISTILLATION DANS UN MODÈLE DE COLONNE UNIQUE

(30) Priority: 24.01.2014 US 201461931440 P
(43) Date of publication of application: 30.11.2016
(73) Proprietor: Sulzer Management AG, 8401 Winterthur (CH)
(72) Inventor: BHARGAVA, Manish, Katy, TX 77494 (US); GENTRY, Joseph, C., Houston, TX 77079 (US)
(74) Representative: Henkel & Partner mbB
(86) International application number: PCT/US2015/012799
(87) International publication number: WO 2015/112927

(56) References cited:
- EP-A1- 2 660 231
- WO-A1-01/79389
- JP-A- H1 057 704
- US-A- 5 709 780
- US-A1- 2005 199 482
- US-A1- 2006 021 911
- US-A1- 2006 287 563
- US-A1- 2007 260 103
- US-A1- 2008 161 618
- US-A1- 2009 139 852
- US-A1- 2010 224 536
- US-B1- 6 348 637
- ANTON A KISS ET AL: "Enhanced bioethanol dehydration by extractive and azeotropic distillation in dividing-wall columns", SEPARATION AND PURIFICATION TECHNOLOGY, ELSEVIER SCIENCE, AMSTERDAM, NL, vol. 86, 18 October 2011 (2011-10-18), pages 70-78, XP028452993, ISSN: 1383-5866, DOI: 10.1016/J.SEPPUR.2011.10.022 [retrieved on 2011-10-25]

## Description

### FIELD OF THE INVENTION

Partial condensers are used in distillation columns when the distillate product is removed as a vapor stream. This approach is commonly employed when there are very light, i.e., volatile components in the feed to the column that would require a high column pressure and a low condenser temperature to completely condense these very volatile components. The use of a partial condenser can avoid the use of costly refrigeration in the condenser. Many distillation schemes with partial condensers have both liquid and vapor products. In such cases there is considerable spillover of heavier components in the vapor product and vice versa. It is not possible to maintain a sharp split between liquid and vapor products in conventional partial condensation schemes. This drawback can be overcome by use of a top dividing wall column, which uses both absorption and distillation techniques on either side of dividing wall to provide a sharp split between vapor and liquid components.

### BACKGROUND OF THE INVENTION

There are several distillation schemes in aromatics complex that use partial condenser for separation of lighter components. Typical examples of such columns include a C₅ stabilizer or a dehepatnizer column.

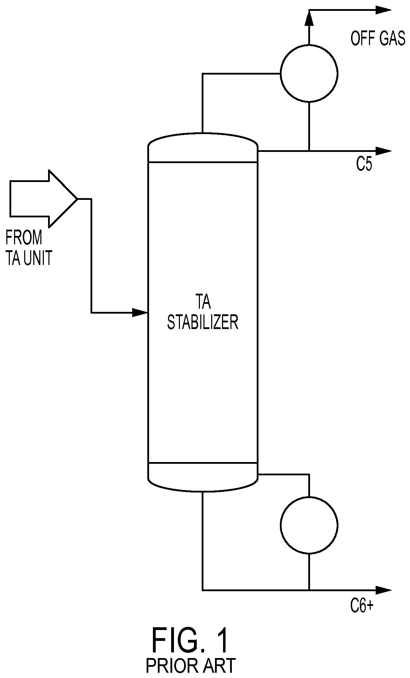

FIG. 1 represents the prior art system of a conventional C₅ stabilizer. This conventional stabilizer aims at separating C₁-C₄ components as an overhead vapor product, C₅ as an overhead liquid product and C₆₊ components as the bottoms product of the column. The column operates at 8.5 kg/cm²g with an overhead temperature of 40°C.

However, the prior art system possesses several disadvantages. It is not possible to condense the lighter components in the overhead product at 8.5kg/cm²g and use cooling water as the overhead cooling media. The overhead system has a partial condenser. Lighter components (used as offgas) are drawn as the vapor product from the partial condenser. C₅ product is the liquid stream from the partial condenser. A considerable amount of C₅ components are lost to the offgas vapor stream. The loss of C₅ components can be prevented by decreasing the overhead temperature (e.g., by using refrigeration) or increasing the column pressure. However, this increases the operating cost of the column. The prior art systems provides a loose split between C₁ to C₄ in vapor and C₅ in liquid product, the recoveries of these components is also low as they are lost in the off gas vapor stream.

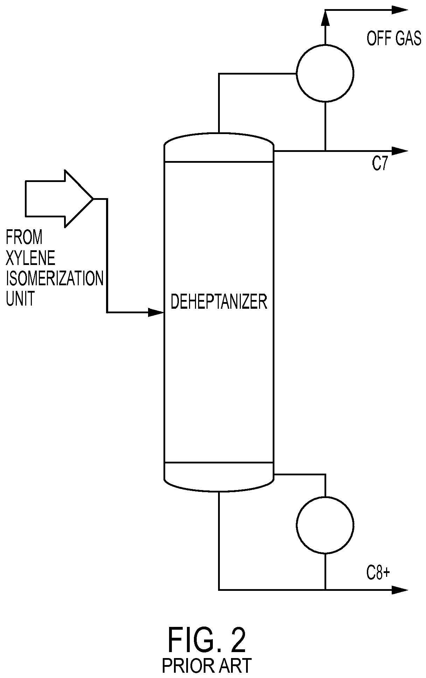

FIG. 2 represents the prior art system of a conventional dehepatnizer column. This conventional dehepatnizer aims at separating lighter components (C₁ to C₅) as an overhead vapor product, C₇ as an overhead liquid product and C₈₊ components as the bottoms product of the column. The column operates at 5.0 kg/cm²g with an overhead temperature of 40°C.

Other prior art is disclosed in US6348637B1 and in Enhanced bioethanol dehydration by extractive and azeotropic distillation in dividing-wall columns by Anton A.Kiss, DavidJ.P.C.Suszwalak, Separation and Purification Technology 86 (2012)70-78.

### SUMMARY OF THE INVENTION

The invention is directed to a process wherein two different unit operations (absorption and distillation) take place on either side of a top dividing wall column. One side of the dividing wall column uses absorption to separate non-condensable components from the feed; the other side of the dividing wall uses distillation to separate heavier liquid components. The column is defined in claim 1 and the associated process is defined in claim 2.

### BRIEF DESCRIPTION OF THE DRAWINGS

FIG. 1 represents a conventional C₅ stabilizer in accordance with the prior art;
FIG. 2 represents a conventional dehepatnizer column in accordance with the prior art;
FIG. 3 represents a process scheme in accordance with an embodiment of the invention for a stabilizer design; and
FIG. 4 represents process scheme in accordance with an embodiment of the invention for a deheptanizer design.

### DETAILED DESCRIPTION OF EXEMPLARY EMBODIMENTS

An embodiment of the invention is directed to a process wherein two different unit operations (absorption and distillation) take place on either side of a top dividing wall column.

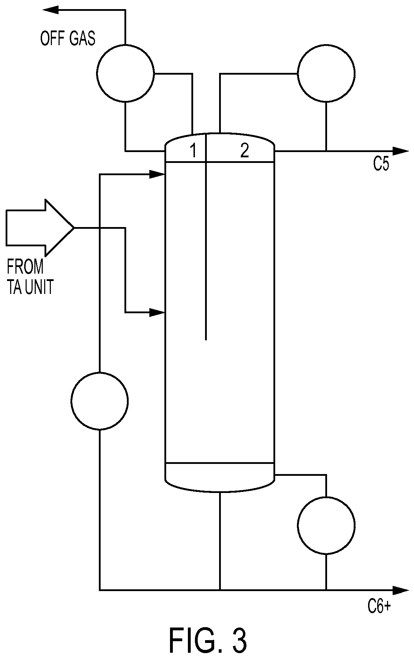

In an embodiment of the invention shown in FIG. 3, the process scheme of the claimed invention is designed to separate light components (non condensable), C₅ (Top liquid product), and C₆₊ (Heavies) in a single top divided column. The feed stream is first sent to the pre-fractionation side of the top divided column. The vertical dividing wall splits the top portion of the column into two halves. The feed side of the wall is called the pre-fractionation section. The non-condensables (used as off gas) is removed as overhead vapor product from a vent condenser. In certain embodiments of the invention, the column overhead pressure is set at 2.7 kg/cm2g via a pressure controller on the overhead vapor product line. The section above the feed acts as an absorption section that is primarily used to minimize the loss of heavier components. The pre-fractionation side has reflux coming from two sources: A liquid stream condensed from a vent condenser; and a heavy stream from a bottoms pump.

In an embodiment of the invention, the vapor from the overhead of the main section is condensed and cooled to 40°C in air-cooled exchanger followed by the water-cooled condenser. The condenser outlet is collected in an overhead receiver. The C₅ liquid is pumped out of the drum via reflux pumps. A portion of the light liquid is sent back to the column as reflux and the remainder is withdrawn as C₅ product.

In an embodiment of the invention, the temperature in top section of the main column is controlled in cascade with the reflux flow control loop. This allows control over the quality of the C₅ product by suppressing the tendency of the heavier components from going to the top of the column.

In an embodiment of the invention, the reboiler connected to the main section is a thermosyphon steam reboiler that uses steam as heating medium. The heat input to the reboiler is regulated by controlling the steam flow cascaded to the column bottom tray temperature controller.

The C₅ bottom product is controlled by a level control loop in cascade with the bottom product flow rate.

Table 1 presents a comparison of operational parametersbetween the conventional stabilizer design and the TDWC stabilizer design of the claimed invention.

**Table 1**

| **Operational Parameters** | **Units** | **Conventional Design** | **Stabilizer design of the invention** |
|---|---|---|---|
| C₅ in product | Kg/hr | 367 | 659 |
| Operating Pressure | Kg/cm²g | 8.3 | 2.7 |
| RVP | Psia | 107 | 27 |
| Overhead temperature | °C | 40 | 40 |
| Duty | MMkcal/hr | 4.7 | 4.7 (integrated with HAC Overhead) |
| C₅ recovery | Wt% | 52.3% | 96.8% |
| C₅ purity | Wt% | 59.1% | 90.4% |
| Benzene in C5 product | Wt% | 0.1% | 0.1% |
| Bottoms temperature | °C | 229 | 183 |

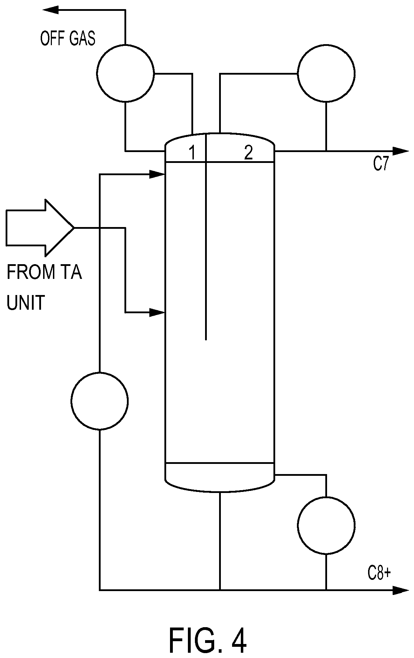

FIG. 4 represents a process scheme in accordance with an embodiment of the invention for a deheptanizer design. The column pressure is reduced to 1.8 kg/cm2g. GT-Low pressure deheptanizer reduces the energy consumption by 19%.

Table 2 presents a comparison between the conventional deheptanizer design and the TDWC stabilizer design of the claimed invention.

**Table 2**

| **Operational Parameters** | **Units** | **Conventional Design** | **Stabilizer design of the invention** |
|---|---|---|---|
| Operating Pressure | Kg/cm²g | 5 | 1.8 |
| Overhead temperature | °C | 40 | 40 |
| Duty | Mmkcal/hr | 23.3 | 18.9 |
| Bottoms temperature | °C | 227 | 193 |

Overall aspects of the invention relate to methods for increasing the energy efficiency or better product purities in a distillation process using a top divided column.

## Claims

1. A distillation column comprising:
a vertical dividing wall splitting a top portion of the column into first and second halves, wherein the first half comprises a pre-fractionation side;
a feedline coupled to the first half and configured to provide feed to the first half;
a pre-fractionation absorption section located in the pre-fractionation side and configured to separate non-condensable components from the feed;
a distillation unit located in the second half and configured to separate heavier liquid components;
a vent condenser being configured to receive overhead product from the pre-fractionation side, to remove non-condensables as overhead vapor product and to reflux a liquid stream condensed in the vent condenser to the pre-fractionation side;
a total condenser coupled to the second half and configured to receive overhead product from the second half;
a bottoms pump and a bottoms feedline coupled to the distillation column, wherein the bottoms pump is configured to receive bottom product from the distillation column and to pump the bottom product as reflux via the bottoms feedline to the pre- fractionation side;
a reboiler coupled to the distillation column and configured to receive bottom product from the distillation column; and
a bottoms removal line configured to remove bottom product from the distillation column.

2. A process of separating non-condensable components and heavier liquid components from a feed stream, the process comprising the steps of:
a. providing a distillation column as claimed in claim 1;
b. providing the feed stream to the pre-fractionation side of the column;
c. subjecting the feed stream in the pre-fractionation side of the column to separation by absorption to separate the non-condensable lighter components from the feed stream;
d. removing the non-condensable lighter components from the pre-fractionation side of the column;
e. subjecting the feed stream on the second side of the column to separation by distillation using an extractive solvent to separate C5 component from the feed stream;
f. removing the C5 component from the second side of the column; and
g. removing the bottom product from the column.

## Patentansprüche

1. Destillationskolonne, umfassend:
eine vertikale Trennwand, die einen oberen Abschnitt der Kolonne in eine erste und eine zweite Hälfte aufteilt, wobei die erste Hälfte eine Vorfraktionierungsseite umfasst;
eine Zuführungsleitung, die mit der ersten Hälfte gekoppelt und dafür konfiguriert ist, der ersten Hälfte eine Zuführung bereitzustellen;
einen Vorfraktionierungs-Absorptionsbereich, der sich auf der Vorfraktionierungsseite befindet und dafür konfiguriert ist, nicht kondensierbare Komponenten von der Zuführung abzutrennen;
eine Destillationseinheit, die sich in der zweiten Hälfte befindet und dafür konfiguriert ist, schwerere flüssige Komponenten abzutrennen;
einen Entlüftungskondensator, der dafür konfiguriert ist, ein Überkopfprodukt von der Vorfraktionierungsseite aufzunehmen, nicht kondensierbare Stoffe als Überkopfdampfprodukt zu entfernen und einen im Entlüftungskondensator kondensierten Flüssigkeitsstrom zu der Vorfraktionierungsseite zurückfließen zu lassen;
einen Gesamtkondensator, der mit der zweiten Hälfte gekoppelt und dafür konfiguriert ist, ein Überkopfprodukt aus der zweiten Hälfte aufzunehmen;
eine Bodenpumpe und eine Bodenzuführungsleitung, die mit der Destillationskolonne gekoppelt sind, wobei die Bodenpumpe dafür konfiguriert ist, das Bodenprodukt von der Destillationskolonne aufzunehmen und das Bodenprodukt als Rückfluss über die Bodenzuführungsleitung zu der Vorfraktionierungsseite zu pumpen;
einen Reboiler, der mit der Destillationskolonne gekoppelt und dafür konfiguriert ist, das Bodenprodukt von der Destillationskolonne aufzunehmen; und
eine Bodenentfernungsleitung, die dafür konfiguriert ist, das Bodenprodukt von der Destillationskolonne zu entfernen.

2. Vorgang zum Trennen nicht kondensierbarer Komponenten und schwererer flüssiger Komponenten von einem Zuführungsstrom, wobei der Vorgang die Schritte umfasst:
a. Bereitstellen einer Destillationskolonne nach Anspruch 1;
b. Bereitstellen des Zuführungsstroms zu der Vorfraktionierungsseite der Kolonne;
c. Unterziehen des Zuführungsstroms auf der Vorfraktionierungsseite der Kolonne einer Abtrennung durch Absorption, um die nicht kondensierbaren leichteren Komponenten vom Zuführungsstrom abzutrennen;
d. Entfernen der nicht kondensierbaren leichteren Komponenten von der Vorfraktionierungsseite der Kolonne;
e. Unterziehen des Zuführungsstroms auf der zweiten Seite der Kolonne der Abtrennung durch Destillation unter Verwendung eines Extraktionslösungsmittels, um die C5-Komponente von dem Zuführungsstrom abzutrennen;
f. Entfernen der C5-Komponente von der zweiten Seite der Kolonne; und
g. Entfernen des Bodenprodukts von der Kolonne.

## Revendications

1. Colonne de distillation comprenant :
une paroi de séparation verticale divisant une partie supérieure de la colonne en première et seconde moitiés, la première moitié comprenant un côté de pré-fractionnement ;
une ligne d'alimentation étant accouplée à la première moitié et étant conçue pour alimenter la première moitié ;
une section d'absorption de pré-fractionnement étant située dans le côté de pré-fractionnement et étant conçue pour séparer les composants non condensables de l'alimentation ;
une unité de distillation étant située dans la seconde moitié et étant conçue pour séparer les composants liquides plus lourds ;
un condenseur d'évacuation étant conçu pour recevoir le produit de tête du côté de pré-fractionnement, pour éliminer les non condensables en tant que produits de vapeur de tête et pour renvoyer un courant liquide condensé dans le condenseur d'évacuation vers le côté de pré-fractionnement ;
un condenseur total étant accouplé à la seconde moitié et étant conçu pour recevoir le produit de tête de la seconde moitié ;
une pompe de fond et une ligne d'alimentation de fond étant accouplées à la colonne de distillation, la pompe de fond étant conçue pour recevoir les produits de fond de la colonne de distillation et pour pomper les produits de fond en tant que reflux par le biais de la ligne d'alimentation de fond vers le côté de pré-fractionnement ;
un rebouilleur accouplé à la colonne de distillation et conçu pour recevoir les produits de fond de la colonne de distillation ; et
une ligne d'élimination des fonds étant conçue pour éliminer les produits de fond de la colonne de distillation.

2. Procédé de séparation de composants non condensables et de composants liquides plus lourds d'un courant d'alimentation, le procédé comprenant les étapes de :
a. prévention d'une colonne de distillation selon la revendication 1 ;
b. fourniture du courant d'alimentation vers le côté de pré-fractionnement de la colonne ;
c. soumission du courant d'alimentation dans le côté de pré-fractionnement de la colonne à une séparation par absorption pour séparer les composants plus légers non condensables du courant d'alimentation ;
d. retrait des composants plus légers non condensables du côté de pré-fractionnement de la colonne ;
e. soumission du courant d'alimentation sur le second côté de la colonne à une séparation par distillation au moyen d'un solvant d'extraction pour séparer le composant C5 du courant d'alimentation ;
f. retrait du composant C5 du second côté de la colonne ; et
g. retrait des produits de fond de la colonne.
